# EUROPÄISCHE PATENTANMELDUNG

(11) **EP 2 484 310 A1**
(43) Veröffentlichungstag der Anmeldung: **08.08.2012**
(21) Anmeldenummer: 12151780.9
(22) Anmeldetag: 19.01.2012
(51) Int. Cl.: A61F 2/24

(54) **Herzklappenprothese mit flexiblen Befestigungen und Implantationsvorrichtung dafür**

(30) Priorität: 08.02.2011 US 201161440425 P
(71) Anmelder: Biotronik AG, 8180 Bülach (CH)
(72) Erfinder: Rzany, Alexander, 90449 Nürnberg (DE); Fringes, Matthias, 91522 Ansbach (DE); Borck, Alexander, 91086 Aurachtal (DE); Schmiedl, Robert, 96114 Hirschaid (DE); Harder, Claus, 91080 Uttenreuth (DE)
(74) Vertreter: Lindner-Vogt, Karin L.

(57) **Zusammenfassung**

Die Erfindung betrifft eine Implantationsvorrichtung (10a-d) mit zumindest einem medizinischen Implantat (12a-d) und zumindest einer Implantationshilfe (14a-d) mit zumindest einem Aufweitelement (16a-d). Es wird vorgeschlagen, dass das Aufweitelement (16a-d) in zumindest einen Hohlraum (18a-d) des Implantats (12a-d) einführbar ist und der zumindest eine Hohlraum (18a-d) mittels des Aufweitelements (16a-d) aufweitbar ist und das Aufweitelement (16a-d) im bestimmungsgemäßen Endzustand zumindest teilweise in dem zumindest einen Hohlraum (18a-d) angeordnet ist, wobei das Aufweitelement (16a-d) als ein Festkörper (20a-d) ausgebildet ist.

## Beschreibung

Die Erfindung betrifft eine Implantationsvorrichtung nach dem Oberbegriff des Patentanspruchs 1 sowie eine Implantationshilfe nach dem Oberbegriff des Patentanspruchs 2, ein Aufweitelement nach dem Oberbegriff des Patentanspruchs 5 und ein medizinisches Implantat zur Implantation in einen tierischen und/oder menschlichen Körper nach dem Oberbegriff des Patentanspruchs 8.

In der Medizin kommen Implantate zum Einsatz, die zur Erfüllung von Ersatzfunktionen permanent oder zumindest für einen längeren Zeitraum in einen tierischen und/oder menschlichen Körper eingebracht werden. Bekannt sind z.B. Herzklappenimplantate, wie etwa Aortenklappenimplantate, welche die Funktion der natürlichen Aortenklappe übernehmen. Hierbei wird das Klappenimplantat nach Expansion der Implantatstruktur sofort nach der Implantation fixiert und nimmt die Position der natürlichen Aortenklappe ein.

Ein häufiges Problem ist hierbei, dass das Implantat mit einer Fehlstellung fixiert wird, was zu einem Versagen des Implantats führen kann. Dies tritt beispielsweise oft bei Kalzifizierung, d.h. der Ablagerung von Kalziumsalzen, insbesondere Kalziumphosphat (Hydroxyapatit) an den Strukturen des Herzens und insbesondere bei einer stark asymmetrisch kalzifizierten Aortenstenose auf. Zudem können oft bereits positionierte Implantate nicht mehr in ihrer Position verändert werden. Des Weiteren kommen häufig Verankerungsmechanismen zum Einsatz, die das Gewebe an einer Implantationsstelle verletzen können.

Der Erfindung liegt die Aufgabe zugrunde, eine Implantationsvorrichtung mit einer Implantationshilfe und einem Aufweitelement sowie einem medizinischen Implantat zu schaffen, mittels der das Implantat exakt, zuverlässig, gewebeschonend und repositionierbar an einer Implantationsstelle implantiert werden kann.

Die Aufgabe wird erfindungsgemäß durch die Merkmale der Patentansprüche 1, 2, 5 und 8 gelöst. Günstige Ausgestaltungen und Vorteile der Erfindung ergeben sich aus den weiteren Ansprüchen, der Zeichnung und der Beschreibung.

Die Erfindung geht aus von einer Implantationsvorrichtung mit zumindest einem medizinischen Implantat und zumindest einer Implantationshilfe mit zumindest einem Aufweitelement.

Es wird vorgeschlagen, dass das Aufweitelement in zumindest einen Hohlraum des Implantats einführbar ist und der zumindest eine Hohlraum mittels des Aufweitelements aufweitbar ist und das Aufweitelement im bestimmungsgemäßen Endzustand zumindest teilweise in dem zumindest einen Hohlraum angeordnet ist, wobei das Aufweitelement als ein Festkörper ausgebildet ist. Durch die erfindungsgemäße Ausgestaltung kann eine Implantationsvorrichtung bereitgestellt werden, mittels der das Implantat optimal positioniert und schonend verankert werden kann. Ferner kann das Implantat so vorteilhaft an die Parameter bzw. an die anatomischen Gegebenheiten der jeweiligen Implantationsstelle, wie die Kalzifizierung einer Blutgefäßwand und/oder eines Annulus, und/oder eine andere, angeborene und/oder krankhafte, Anomalie der Implantationsstelle, angepasst werden. Des Weiteren erlaubt sie eine optimale Platzierung an der Stelle der defekten physiologischen Einheit, wie beispielsweise einer Klappe. Zudem ist eine Repositionierung und dauerhafte Fixierung des Implantats *in vivo* ermöglicht.

In diesem Zusammenhang soll unter einer "Implantationsvorrichtung" insbesondere eine Vorrichtung, wie beispielsweise ein Kathetersystem, verstanden werden, mittels der ein Implantat zu einer temporären oder permanenten Implantation in einen tierischen und/oder menschlichen Körper eingebracht wird. Unter einem "Implantat" soll insbesondere ein Körper verstanden werden, der zumindest eine Ersatzfunktion permanent, für einen längeren Zeitraum oder temporär bei Implantation in den tierischen und/oder menschlichen Körper erfüllt. Denkbar wären hier sämtliche, dem Fachmann als zweckdienlich erscheinenden medizinischen Implantate, wie beispielsweise ein Herzschrittmacher, ein Hirnschrittmacher, ein Herzimplantat, ein Cochleaimplantat, ein Retinaimplantat, ein zahnmedizinisches Implantat, ein Implantat zum Gelenkersatz, eine Gefäßprothese oder andere. Besonders vorteilhaft wird eine Ausbildung des medizinischen Implantats als ein Klappenimplantat vorgeschlagen. Weiterhin ist auch eine Ausgestaltung als ein Sicherheitsimplantat denkbar, das während eines Eingriffs beispielsweise als Rückhaltemittel im Bereich des Eingriffs implantiert wird.

Eine "Implantationshilfe" stellt hier insbesondere ein Mittel, wie beispielsweise einen Katheterschaft, eine Kanüle oder insbesondere einen Draht und/oder eine Feder, dar, das bei der Implantation zur Hilfe genommen wird. Unter einem "Aufweitelement" soll hier insbesondere ein Element verstanden werden, das durch Einnahme einer bestimmten Konfiguration und/oder Form ein anderes Element in dessen Ausdehnung ändert. Ein Aufweiten erfolgt insbesondere durch ein Vorschieben des Aufweitelements in den Hohlraum. Hierdurch und insbesondere durch ein langsames Vorschieben kann vorteilhaft eine gute Anpassung des Implantats an eine Herzmorphologie realisiert werden. Ferner kann die Aufweitung des Hohlraums durch ein Zurückziehen des Aufweitelements wieder reduziert werden, was eine einfache Möglichkeit einer Repositionierung bietet.

Ein "Hohlraum" stellt hier insbesondere einen Raum dar, der eine Ausnehmung aufweist, die zumindest zu 90%, bevorzugt zu zumindest 95% und besonders bevorzugt zu zumindest 98% von einer Umhüllung umgeben ist. Ein "bestimmungsgemäßer Endzustand" ist hier insbesondere der implantierte Zustand. Unter "zumindest teilweise" sollen hier bevorzugt mehr als 50%, besonders vorteilhaft mehr als 75% und besonders vorteilhaft mehr als 95% des Aufweitelements verstanden werden. Bevorzugt sind 100% des Aufweitelements, also das komplette Element, im Hohlraum angeordnet.

In diesem Zusammenhang soll unter einem "Festkörper" insbesondere ein Körper in seinem festen Aggregatzustand verstanden werden. Bevorzugt ist der Festkörper ein elastischer Festkörper, wobei unter "elastisch" verstanden werden soll, dass der Festkörper reversibel auslenkbar ist und/oder eine Konfiguration einnehmen kann, bei der er eine Federkonstante aufweist. Unter einem Festkörper soll hier insbesondere kein Gas, keine Flüssigkeit und/oder kein Gel verstanden werden. Der Festkörper kann aus einem Material wie einem Polymer, Glas, Gummi, Metall, einer Keramik, einer Legierung und/oder jedem anderen, vom Fachmann als sinnvoll erachteten Material gebildet sein. Ferner kann der Festkörper jede dem Fachmann als zweckdienlich erscheinende Gestalt bzw. Form, wie beispielsweise ein linearer Draht, eine Feder und/oder Spiralfeder, aufweisen.

Ferner geht die Erfindung aus von einer Implantationshilfe mit zumindest einem Zuführelement und zumindest einem Aufweitelement für eine Implantationsvorrichtung.

Es wird vorgeschlagen, dass die Implantationshilfe zumindest eine lösbare Verbindung zwischen dem zumindest einen Zuführelement und dem zumindest einen Aufweitelement aufweist. Durch die erfindungsgemäße Ausgestaltung kann eine Manipulation des Aufweitelements mittels des Zuführelements mehrfach und selbst nach einer bereits erfolgten Positionierung im Hohlraum durch erneutes Verbinden der Elemente erfolgen. Ferner kann eine Zu- und Einführung des Aufweitelements in den Hohlraum konstruktiv einfach realisiert werden.

In diesem Zusammenhang soll unter einer "lösbaren Verbindung" insbesondere eine reversible Verbindung bzw. eine Verbindung verstanden werden, die reversibel öffenbar und schließbar bzw. zu öffnen bzw. zu schließen ist. Diese Verbindung kann mittels jeder vom Fachmann für sinnvoll erachteten Verbindungsart, wie beispielsweise eine Schraubverbindung, eine Klettverbindung, eine Steckverbindung, einer Drehverbindung, einem Formschluss, einem Kraftschluss, realisiert sein. Unter einem "Zuführelement" soll hier insbesondere ein Element verstanden werden, das während der Implantation mit dem Implantat der Implantationsstelle und/oder dem sich schon an der Implantationsstelle befindlichen Implantat zugeführt wird. Das Zuführelement wird nicht implantiert. Das Zuführelement und das Aufweitelement sind bevorzugt aus demselben Material gefertigt. Grundsätzlich ist jedoch auch die Wahl unterschiedlicher, dem Fachmann für sinnvoll erscheinender Materialien denkbar.

Eine konstruktiv einfache und zuverlässige lösbare Verbindung kann vorteilhaft bereitgestellt werden, wenn die lösbare Verbindung einen Bajonettverschluss aufweist. Hierbei ermöglicht ein Drehen des Zuführelements gegen den Uhrzeigersinn eine Verbindung bzw. ein Schließen der Verbindung wischen Zuführelement und Aufweitelement. Außerdem ist eine Rotation gegen den Uhrzeigersinn um die eigene Achse möglich, was das Vorschieben des Aufweitelements in den Hohlraum vorteilhaft unterstützen kann. Ein Drehen im Uhrzeigersinn wiederum öffnet die Verbindung.

Alternativ kann es vorteilhaft sein, wenn an dem Zuführelement, insbesondere an seinem distalen Ende, Spannbacken für die lösbare Verbindung angeordnet sind. Hierbei weist das Aufweitelement an seinem proximalen Ende ein Gegenstück, wie beispielsweise einen Vierkant, auf. In diesem Zusammenhang soll unter einem "proximalen Ende" ein Ende verstanden werden, das im Implantationsprozess zum Bediener bzw. zum Arzt weist. Dementsprechend ist ein "distales Ende" ein Ende, das vom Bediener weg weist. Die Spannbacken lassen sich beispielsweise durch eine Drehbewegung des Zuführelements öffnen bzw. schließen. Dadurch wird eine zuverlässige mechanische Verbindung hergestellt, welche sowohl Drehbewegungen als auch lineare Bewegungen in beiden Richtungen erlaubt.

Vorteilhafterweise ist das Zuführelement von einem elastischen Draht gebildet. Dieser Draht ist vorzugsweise aus einer Ni-Ti-Legierung, wie Nitinol, medizinischem Edelstahl, wie der Legierung 316L, CoCr-Legierungen MP35N und L605 und/oder einer Pt/Ir-Legierung gefertigt. Grundsätzlich wäre jedoch auch jedes andere, dem Fachmann für anwendbar erscheinendes Material, wie Kunststoff, Glas, Keramik und/oder Gummi, denkbar. Durch den elastischen Draht kann eine Zuführung zu der Implantationsstelle konstruktiv einfach und Platz sparend realisiert werden. Zudem wird beispielsweise ein Blutfluss in einem bei der Implantation benutztem Gefäßsystem nur minimal gestört.

Des Weiteren geht die Erfindung aus von einem Aufweitelement mit einem sich zwischen einem distalen Ende und einem proximalen Ende erstreckenden Körper, für eine Implantationsvorrichtung.

Es wird vorgeschlagen, dass an dem distalen Ende eine Verbindungsstruktur zur Verbindung mit einem Fixiermittel eines Implantats und/oder an dem proximalen Ende eine Verbindungsstruktur zur Verbindung mit einem Zuführelement einer Implantationshilfe angeordnet ist. Ein "Körper" stellt hier insbesondere das Aufweitelement selbst dar. In diesem Zusammenhang soll unter einer "Verbindungsstruktur" insbesondere eine Struktur verstanden werden, die zwei Elemente permanent oder bevorzugt reversibel miteinander verbindet. Die Verbindungsstruktur am distalen Ende ist vorteilhaft so ausgestaltet, dass sie unverlierbar mit einem Gegenstück verbunden werden kann und ist bevorzugt von einem Vierkant gebildet. Die Verbindungsstruktur am proximalen Ende geht insbesondere eine reversible Verbindung ein und ist bevorzugt als Teil eines Bajonettverschlusses oder als Vierkant, der mit Spannbacken interagieren kann, ausgebildet. Grundsätzlich wäre auch jede andere, dem Fachmann als sinnvoll erscheinende Struktur denkbar. Bevorzugt ist an beiden Enden des Aufweitelements eine Verbindungsstruktur angeordnet. Mittels der Verbindungsstruktur am distalen Ende kann ein Lösen vom Zuführelement konstruktiv einfach erfolgen. Durch die Verbindungsstruktur am proximalen Ende kann ein Mitdrehen des Aufweitelements einfach verhindert werden, was ein sicheres Funktionieren des Aufweitens und damit eine exakte Platzierung und Fixierung des Implantats gewährleistet.

Zudem kann es vorteilhaft sein, wenn der Körper von einem elastischen Draht gebildet ist. Der Draht kann aus denselben Materialien gefertigt sein, wie der Draht des Zuführelements und ist insbesondere ein Metalldraht aus Nitinol. Durch die Ausführung als elastischer Draht kann das Aufweiten konstruktiv einfach erfolgen. Ferner kann das Aufweiten durch Zurückziehen des Drahtes schnell und unkompliziert rückgängig gemacht werden, was eine Repositionierung des Implantats vereinfacht. Zudem kann wegen der wiederherstellbaren Verbindung zum Zuführelement die Repositionierung jederzeit erfolgen.

Eine bevorzugte Weiterbildung besteht darin, dass der Körper dazu vorgesehen ist, als eine Feder mit ausgeprägten Radialkräften zu wirken. Durch das Vorschieben des Aufweitelements in den Hohlraum nimmt der Draht durch die Führung an einer Wand des Hohlraums eine elliptische Form an. Der Draht wird dadurch reversibel zu einer Feder bzw. Ringfeder umgeformt, welche Radialkräfte ausübt und den Hohlraum ausdehnt. Dadurch wird eine Fixierung des Implantats an der Implantationsstelle erreicht. Hierbei kann der Draht im Hohlraum mehrere Windungen machen. Ferner lassen sich die mechanischen Eigenschaften über das konkrete Design an die Anwendung anpassen. Durch dieses Ringfederprinzip und dessen Art der Fixierung des Implantats ist eine selbständige bzw. automatische Anpassung an anatomische Gegebenheiten einfach und vorteilhaft realisierbar.

Zudem geht die Erfindung aus von einem medizinischen Implantat zur Implantation im tierischen und/oder menschlichen Körper mit einem Grundkörper, der zumindest ein Element mit zumindest einem Hohlraum aufweist, für eine Implantationsvorrichtung.

Es wird vorgeschlagen, dass der Hohlraum dazu ausgebildet ist, im bestimmungsgemäßen Endzustand ein Aufweitelement aufzunehmen und zumindest ein Bereich des zumindest einen Elements dazu ausgebildet ist, mit dem Aufweitelement aufweitbar ausgestaltet zu sein, wobei das Aufweitelement im bestimmungsgemäßen Endzustand zumindest teilweise in dem zumindest einen Hohlraum angeordnet ist und als ein Festkörper ausgebildet ist. Durch die erfindungsgemäße Ausgestaltung kann ein Implantat bereitgestellt werden, das gute Selbstpositionierungseigenschaften aufweist und damit eine optimale Platzierung erfährt. Ferner kann ein Druckgradient des auf das medizinische Implantat wirkenden Fließmediums homogen gehalten werden, was vorteilhaft zu einer geringen Materialbelastung beispielsweise bei Klappenimplantaten und dadurch zu einem geringen Ermüdungsrisiko durch gleichmässiges Öffnen der Klappe führt. Dies wiederum resultiert in einer langen Lebensdauer der Segel und damit der Klappe. Des Weiteren können durch die bessere Funktionalität der Klappe, die so einem höheren Druckgradienten standhalten kann, bei asymmetrisch kalzifiziertem Annulus bessere klinische Ergebnisse gegenüber den herkömmlichen Klappenimplantaten erzielt werden. Durch die erfindungsgemäße Ausgestaltung kann ferner eine Symmetrie in der Flussdynamik des Fließmediums erhöht werden, was das weitere Kalzifizierungsrisiko vorteihaft verringert. Ferner ist das Implantat strömungsmechanisch optimiert, wodurch turbulente Strömungen des Fließmediums reduziert werden können, was wiederum in einer geringeren Neigung zur Thrombenbildung resultiert.

Ferner soll in diesem Zusammenhang unter einem "Grundkörper" insbesondere eine Struktur verstanden werden, die im Wesentlichen eine Gestalt und/oder eine Form des Implantats bildet. Der Grundkörper weist bevorzugt zumindest einen Ring bzw. einen hohlen Ring und/oder einen Hohlzylinder, wie beispielsweise einen schlauchförmigen Polymerzylinder, auf. Das Element kann vom Grundkörper selbst gebildet sein oder nur ein Teil des Grundkörpers darstellen. Der Bereich des Elements kann der Hohlraum selbst und/oder dessen Wand bzw. Umhüllung sein. Unterstützend kann das Implantat expandierbar bzw. mittels einer von außen zugeführten Kraft expandierbar bzw. plastisch verformbar sein. Konstruktiv einfach kann die passive Expansion mittels eines Ballonkatheters erfolgen, wobei das Implantat auf einen Ballonkatheter crimpbar sein kann.

Ferner wird vorgeschlagen, dass das Implantat zumindest ein Führungsmittel aufweist. Unter einem "Führungsmittel" soll insbesondere ein Mittel verstanden werden, das mittels einem seiner Parameter, wie eine Orientierung, einer Form und/oder einer Anordnung, eine Bewegungsrichtung, einen Weg, eine Ausrichtung und/oder eine Anordnung das Aufweitelements beeinflusst und/oder vorgibt. Vorteilhafterweise ist das Führungsmittel dazu vorgesehen, das Aufweitelement beim Einführen und/oder Aufweiten des Bereichs tangential zu führen, wodurch Vorschubkräfte minimiert werden können. Mittels des Führungsmittels kann eine Bewegung des Aufweitelements vorteilhaft vorbestimmt und somit beeinflusst werden.

Ferner ist das Führungsmittel vorteilhaft an jeweils einem proximalen Ende einer Struktur des Implantats, wie dem Grundkörper, angeordnet, wodurch eine gute Zugänglichkeit des Aufweitelements beim Einführen in den Hohlraum und/oder des Zuführelements beim Kontaktieren des Aufweitelements erreicht werden kann. Vorteilhaft für die Wegfindung ist das Führungsmittel direkt bzw. unmittelbar an einem Zugang, wie einer Aussparung, zu dem Hohlraum angeordnet. Der Zugang bzw. die Aussparung kann ein Verschlussmittel aufweisen, das beim Einführen des Aufweitelements überwunden wird. Ferner kann vorteilhaft verhindert werden, dass beispielsweise Luft aus dem Hohlraum entweichen kann und/oder dass Blut in den Hohlraum eindringen kann, wenn in der Aussparung als Verschlussmittel beispielsweise ein Ventil angeordnet ist, das einen ungewollten Medienaustausch verhindert. Zudem kann es vorteilhaft sein, wenn im bestimmungsgemäßen Endzustand die Aussparung durch einen passgenauen Sitz des proximalen Endes des Aufweitelements verschlossen werden kann, wodurch Bauteil sparend auf ein separates Verschlussmittel verzichtet werden kann. Generell wäre es auch möglich, dass das proximale Ende über eine Außenoberfläche des Grundkörpers hinaus ragt. Besonders bevorzugt ist das Führungsmittel von einem Trichter gebildet. In einer alternativen Realisierung kann das Führungsmittel bzw. der Trichter am distalen Ende des Zuführelements angeordnet sein, wodurch im implantierten Zustand von dem nun fehlenden Führungsmittel keine Verwirbelungen des Bluts entstehen können. Alternativ ist auch eine nachträgliche minimalinvasive Entfernung des Trichters vom Implantat möglich, z.B. während einer Follow-Up-Untersuchung.

Eine gute Verankerung des Aufweitelements mit einer hohen Haltekraft kann vorteilhaft erreicht werden, wenn das Implantat zumindest ein Fixiermittel aufweist. Das Fixiermittel ist vorteilhafterweise in dem zumindest einen Hohlraum und bevorzugt an einem Ende des durch das Führungsmittel vorbestimmten Wegs des Aufweitelements angeordnet, wodurch das Aufweiten des Hohlraums durch das Aufweitelement kontrolliert ablaufen kann. Die Anordnung des Fixiermittels erfolgt bevorzugt in einer Wand, deren Ebene sich im Wesentlichen senkrecht bzw. senkrecht zu der Bewegungsrichtung bzw. Vorschubrichtung des Aufweitelements erstreckt. Um mehrere Windungen des Aufweitelements im Hohlraum zuzulassen, kann diese Wand Aussparungen für einen Durchtritt des Aufweitelements aufweisen. Ferner ist das Fixiermittel dazu vorgesehen, das Aufweitelement zumindest im bestimmungsgemäßen Endzustand zu fixieren. Eine besonders gute Fixierung kann erfolgen und ein Mitdrehen des Aufweitelements mit dem Zuführelement kann verhindert werden, wenn das Fixiermittel als ein Gegenstück zu einem Vierkant ausgebildet ist. Generell wäre jedoch jedes andere, dem Fachmann für verwendbar erscheinende Fixiermittel denkbar.

In einer weiteren Ausgestaltung der Erfindung wird vorgesehen, dass der Grundkörper zumindest ein Positioniermittel aufweist. Das Positioniermittel kann den gesamten Grundkörper bilden oder auch nur ein Teil des Grundkörpers sein. Somit kann das Positioniermittel eine Verdickung, ein Wulst und/oder eine Ringstruktur sein, die bzw. der an den Grundkörper angeformt und/oder in diesem ausgeformt ist. Grundsätzlich kann das Positioniermittel auch mittels einer anderen Art, wie einem Kraft-, Stoff- oder Formschluss, mit dem Grundkörper verbunden sein. Das Positioniermittel dient insbesondere einer Unterstützung einer Fixierung des Implantats, wodurch vorteilhaft auf beispielsweise domartige Verankerungsmechanismen gemäß des Stands der Technik, die das Gewebe reizen und Entzündungen hervorrufen können, verzichtet werden kann. Zudem kann das Positioniermittel zu einer exakten Positionierung des Implantats verwendet werden. Ferner stellt das Positioniermittel ein Mittel zur mechanischen Verstärkung der Struktur des Implantats dar.

In einer weiteren Ausgestaltung der Erfindung wird vorgeschlagen, dass das Element des Grundkörpers und/oder das Positioniermittel von zumindest einer Ringstruktur gebildet ist, wodurch das Implantat durch eine Selbstpositionierung physiologisch optimal am Annulus platziert und fixiert werden kann. Auch hier kann das Element bzw. die Ringstruktur den gesamten Grundkörper bilden oder auch nur ein Teil des Grundkörpers sein. Die Ringstruktur kann von einem hohlen Ring gebildet sein, wobei die Wand bzw. Umhüllung des Rings dazu ausgebildet ist, durch das Aufweitelement aufgeweitet zu werden und/oder das Aufweitelement bei der Aufweitung zu führen. Die Umhüllung ist insbesondere aus einem flexiblen Polymermaterial gefertigt, wie bevorzugt Polyethylen (PET), Polykarbonat (PC), Polyvinylchlorid (PVC), Polyimid (PI), PEBAX®, PVP, PA11. Grundsätzlich kann es auch aus jedem anderen, dem Fachmann als verwendbar erscheinendem Material gefertigt sein. Durch die Realisierung als Implantat aus Polymer kann dieses extrem flexibel gestaltet werden. Des Weiteren kann ein geringer Implantationsdurchmesser realisiert werden, da sich zum Zeitpunkt der Implantation kein oder wenig Metall im Katheter befindet. Ferner wird durch den fehlenden direkten Kontakt von Metallen mit der Herzwand eine Störung der elektrischen Erregungsausbreitung am Herzen vermieden.

Vorteilhafterweise weist das medizinische Implantat zumindest zwei Ringstrukturen auf, wodurch eine Platzierung und Selbstpositionierung an der Implantationsstelle, wie dem Annulus, besonders exakt erfolgen kann. Eine genaue und sichere Fixierung kann vorteilhaft erreicht werden, wenn die Ringstruktur an einem proximalen Ende und/oder einem distalen Ende des Grundkörpers angeordnet ist. Bevorzugt ist je eine Ringstruktur an dem proximalen Ende und an dem distalen Ende angeordnet, wodurch die Gestalt des Grundkörpers besonders zuverlässig an die Form der Implantationsstelle bzw. dem Annulus angepasst werden kann. Zudem kann so eine komfortable kurze Bauhöhe des Implantats bzw. des Klappenimplantats ermöglich werden, wodurch Gefahren, z.B. die Koronararterieneingänge zu behindern oder zu verschließen, minimiert werden.

Sind die zwei Ringstrukturen unabhängig voneinander ausdehnbar, kann die Positionierung des Implantats besonders präzise erfolgen. Hierbei wird zuerst der distale Ring gedehnt, wodurch ein Anschlag zur optimalen Positionierung des Implantats, beispielsweise in der Form einer künstlichen Aortenklappe, gebildet wird. Erst nach korrekter Platzierung des distalen Rings wird der proximale Ring gedehnt und das Implantat bzw. die Herzklappe automatisch im Bereich der durch die defekte physiologische Herzklappe gebildeten Engstelle zentriert. Eine Repositionierung ist durch Zurückziehen der Drähte möglich.

Zudem wird vorgeschlagen, dass die Ringstruktur mit einem aushärtbaren Material befüllbar ist, wodurch eine dauerhafte Fixierung konstruktiv einfach unterstützt werden kann. Hierbei kann das aushärtbare Material im Befüllungszustand flüssig oder viskos sein. In diesem Zusammenhang soll unter "aushärtbar" insbesondere verstanden werden, dass das Material zum Befüllen, von einem ersten, flüssigen oder weniger viskosen Zustand in einen zweiten, höher viskosen, bevorzugt festen Zustand übergeht. Der Zustandsübergang kann von jedem dem Fachmann als sinnvoll erscheinenden physikalischen, chemischen oder elektrischen Faktor, wie beispielsweise Zeit, Temperatur, Strahlung (IR-, VIS-, UV-, gamma-, radioaktive Strahlung), Ultraschall, Magnetismus, Strom, pH-Wert-, Konzentrations- und/oder Ladungsänderung abhängig sein. Das Material wird vorteilhafterweise stärker polymerisiert und/oder vernetzt. Es kommt vorteilhaft eine vernetzbare Polymerlösung zum Einsatz. Bevorzugt kann sich die Polymerlösung dabei aus mehreren Komponenten zusammensetzen, deren relatives Verhältnis und Zusammensetzung eine gezielte Einstellung physikalischer Eigenschaften vorteilhaft erlaubt. Optional können hierbei auch unterschiedliche Eigenschaften für die einzelnen Ringstrukturen realisiert werden. Vorteilhaft lässt sich über das Verhältnis von polymeren zu monomeren Anteilen sowie die Konzentration von chemisch aktivierenden Substanzen (Initiatoren für die Polymerisation) unmittelbar die Reaktionsgeschwindigkeit beeinflussen. Vorteilhafterweise ist das aushärtbare Material ausgewählt aus einer Gruppe bestehend aus: Acrylate, Methacrylate, Cyanacrylate, Epoxide, Urethane, Acrylamide und/oder Acylsäuren.

Es wird zudem vorgeschlagen, dass zwischen zwei Ringstrukturen zumindest eine Verbindungsstruktur angeordnet ist. In diesem Zusammenhang soll unter einer "Verbindungsstruktur" insbesondere eine Struktur verstanden werden, die die beiden Ringstrukturen mechanisch miteinander verbindet. Sie wird bevorzugt von einer durchgängigen Polymerfolie gebildet, die die defekten humanen Klappen vom neuen, zumeist tierischen, Klappenmaterial des Implantats isoliert, wodurch eine Kontaktfläche zwischen der künstlichen Klappe und einer Aortenwand gewebeschonend minimiert wird. Ferner kann dadurch effektiv eine Kalzifizierung des tierischen Klappenmaterials verhindert werden, die bei direktem Kontakt von den defekten humanen Klappen auf die tierischen Klappen überspringen könnte.

Ferner wird vorgeschlagen, dass der Grundkörper einen Stent umfasst und/oder die Verbindungsstruktur einen Stent bildet. Hierfür kann das Polymer der Verbindungsstruktur aus einem aushärtbaren Material gefertigt sein. Bevorzugt ist es mittels desselben Mechanismus aushärtbar, wie das Material zum Befüllen der Ringstruktur. Dadurch, dass der Grundkörper einen Stent umfasst, kann eine Positionierung des Implantats und/oder eine Anpassung an anatomische Gegebenheiten an der Implantationsstelle weiter unterstützt werden.

Weist das Implantat eine Klappe auf, die in einer axialen Richtung proximal über der Ringstruktur an einem proximalen Ende des Grundkörpers angeordnet ist, kann vorteilhaft ein geringer Implantationsdurchmesser realisiert werden. Hierbei ist die Klappe bevorzugt an der proximalen Ringstruktur befestigt. Grundsätzlich kann die Klappe jedoch auch axial zwischen zwei Ringstrukturen angeordnet sein. Dies resultiert in einer besonders zuverlässigen Justierung der Klappe. Bei Positionierung zwischen den Ringstrukturen kann die Klappe auch an der Verbindungsstruktur angebracht sein und/oder mit einer Ringstruktur oder beiden Ringstrukturen verbunden sein. Die Klappe kann jeweils mittels jeder dem Fachmann für sinnvoll erachtete Verbindungsart, wie beispielsweise Nähen und/oder Kleben mit der Ringstruktur oder der Verbindungsstruktur verbunden sein.

Eine weitere Ausgestaltung der Erfindung sieht vor, dass zumindest an einer Ringstruktur an dem proximalen Ende des Grundkörpers und/oder an einem proximalen Ende einer Klappe eine Stützstruktur angeordnet ist. Diese Stützstruktur dient bevorzugt der Stützung, Positionierung und der proximalen mechanischen Fixierung der Klappe. Sie ist beispielsweise ausgeführt als Metallgerüst in der Form eines Drahtgeflechts bevorzugt aus Nitinol und/oder als eine weitere bzw. dritte Ringstruktur, die unabhängig von den anderen Funktionseinheiten des Implantats positionierbar, aufweitbar und fixierbar ist. Durch die Stutzstruktur kann die relative Positionierung der Ringstrukturen und der Klappensegel sichergestellt werden, um ein exaktes und komplikationsloses Öffnungs- und/oder Schließverhalten der Segel zu gewährleisten. Die Ausführung als weitere Ringstruktur ist insbesondere dann von Vorteil, wenn die diese Ringstruktur eine hohe mechanische Flexibilität aufweist und dadurch eine gute Adaption an die physiologischen Gegebenheiten des Herzen erlaubt. Dadurch können optimale Strömungseigenschaften und eine geringe mechanische Einschränkung der Herzbewegung erreicht werden.

Eine bevorzugte Weiterbildung besteht darin, dass zumindest zwei Ringstrukturen im bestimmungsgemäßen Endzustand in Fließrichtung eines Fließmediums axial vor und hinter einem Annulus angeordnet sind. Unter einer "Fließrichtung eines Fließmediums" soll hier insbesondere die wissenschaftlich bekannte Fließrichtung von arteriellem und/oder venösem Blut im Herzen und besonders vorteilhaft im Falle der Aortenklappe der Fluss von Blut vom linken Ventrikel in die Aorta verstanden werden. Hierbei handelt es sich bei dem Annulus bevorzugt um den Aortenannulus. Durch die Realisierung der erfindungsgemäßen Anordnung kann das Implantat besonders gut an die Anatomie des Herzens bzw. einer Herzklappenregion mit beispielsweise einem Aortenbulbus angepasst werden.

Ferner ist es vorteilhaft, wenn der Grundkörper und/oder die Verbindungsstruktur dazu vorgesehen sind, einen Unterschied in einer Form eines Innenquerschnitts des Grundkörpers/der Verbindungsstruktur und einer Querschnittsfläche einer Implantationsstelle auszugleichen. Unter "vorgesehen" soll insbesondere speziell ausgestattet, ausgelegt und/oder vorbereitet verstanden werden. In diesem Zusammenhang soll unter einer "Form des Innenquerschnitts des Grundkörpers" insbesondere eine weitestgehend runde bzw. zylinderförmige Form verstanden werden, so dass sich Segel der Klappe komplikationslos öffnen und schließen können. Unter einer "Querschnittsfläche einer Implantationsstelle" soll hier insbesondere eine stark asymmetrisch bzw. unrunde Stelle insbesondere mit beispielsweise einer kalzifizierten Aortenstenose verstanden werden. Der Grundkörper passt somit die mögliche uneinheitliche Form eines Außendurchmessers des Grundkörpers vorteilhaft an die Querschnittsfläche der Implantationsstelle an, wodurch das Implantat in besonderem Maße die örtlichen Gegebenheiten an der Implantationsstelle berücksichtigt. Dadurch kann vorteilhaft eine Unsymmetrie der Blutgefäßwand bzw. des Annulus ausgeglichen werden und dennoch eine weitestgehend runde, symmetrische Innenform des Grundkörpers zur benötigten fehlerfreien und komplikationslosen Funktion von Segeln der Klappe beibehalten werden.

Eine vorteilhafte Weiterbildung besteht in einer Ausgestaltung des Implantats als eine Schutzstruktur. In diesem Zusammenhang soll unter einer "Schutzstruktur" insbesondere eine Struktur verstanden werden, die temporär, d.h. beispielsweise über den Zeitraum eines medizinischen Eingriffs, implantiert wird und/oder die während eines Eingriffs eine Schutzfunktion ausüben kann. Die Schutzstruktur ist vorzugsweise als Rückhaltemittel ausgeführt. Hierfür ist an ihrem ringförmigen Grundkörper, bevorzugt an dessen distalem Ende, ein von einem Polymernetz gebildeter Schirm angeordnet. Generell kann der Schirm bzw. das Netz auch aus jedem anderen, vom Fachmann für anwendbar erachteten Material, wie Metall, Gummi, Keramik, ausgeführt sein. Mittels der Ausgestaltung als Schutzstruktur in Verbindung mit dem Ringfederprinzip der reversiblen Aufweitung kann ein Implantat bereitgestellt werden, das schnell, gewebeschonend und sicher implantiert, aber auch wieder unkompliziert explantiert werden kann.

Vorteilhafterweise ist eine Ausgestaltung des Implantats als ein Klappenimplantat. In diesem Zusammenhang soll unter einem "Klappenimplantat" insbesondere ein Körper verstanden werden, der zumindest eine Ersatzfunktion eines Rückschlagventils, permanent oder für einen längeren Zeitraum bei Implantation erfüllt. Denkbar wären hier sämtliche, dem Fachmann als zweckdienlich erscheinende medizinische Klappenimplantate, die in einen Hohlraum, wie beispielsweise einem Verdauungstrakt, einem Bronchialtrakt und/oder einem Blutgefäß, wie insbesondere einer Arterie und/oder einer Vene, des Körpers eingebracht wird. Das Klappenimplantat stellt bevorzugt ein Herzklappenimplantat mit einer Klappe aus natürlichem und/oder künstlichem Material dar.

Eine bevorzugte Weiterbildung ist eine Ausgestaltung des Implantats als Aortenklappe, wodurch eine ausgefeilte Ersatzstruktur für die am häufigsten mit Fehlfunktionen behaftete Herzklappe bereitgestellt werden kann. Auch können Komplikationen wie z.B. Störungen der Mitralklappe oder eine Notwendigkeit eines Herzschrittmachers günstigerweise reduziert werden. Ebenso ist eine Ausgestaltung als Pulmonalklappe, Trikuspidalklappe oder auch eine Ausgestaltung als Mitralklappe denkbar.

Vorteilhaft kann am Implantat eine ablagerungshemmende, insbesondere kalzifizierungshemmende, Beschichtung vorgesehen sein, insbesondere Homocysteinsäure. Damit kann die Gefahr eine Störung oder ein Funktionsausfall des Klappenimplantats weiter verringert werden.

Die Erfindung ist nachfolgend beispielhaft, anhand von einem in Zeichnungen dargestelltes Ausführungsbeispiel näher erläutert. Es zeigen:
- Fig. 1A: eine erfindungsgemäße Implantationsvorrichtung während einer Implantation in einer schematischen Darstellung,
- Fig. 1B: die Implantationsvorrichtung der Fig. 1A bei einer Aufweitung eines Hohlraums eines Implantats in einer schematischen Darstellung,
- Fig. 2: eine lösbare Verbindung zwischen einem Zuführelement und einem Aufweitelement ausgeführt als ein Bajonettverschluss,
- Fig. 3A: eine alternative lösbare Verbindung zwischen einem Zuführelement und einem Aufweitelement mit Spannbacken und einem Vierkant,
- Fig. 3B: die Verbindung der Fig. 3A in einem geschlossenen Zustand,
- Fig. 4: das Implantat im Implantierten Zustand an einem Annulus in einer schematischen Darstellung,
- Fig. 5: eine schematische Darstellung des Einführens der Implantatvorrichtung der Fig. 1 mit dem Implantat an einer Implantationsstelle,
- Fig. 6: eine schematische Darstellung eines Schnitts entlang der Linie VI-VI der Fig. 5 durch eine Aortenwand mit Aufsicht auf Segel des Implantats,
- Fig. 7: eine Ringstruktur des Implantats der Fig. 4 mit einem Führungsmittel,
- Fig. 8: eine vergrößerte Darstellung des Führungsmittels der Fig. 7 mit dem Bajonettverschluss des Aufweitelements,
- Fig. 9: ein alternatives Implantat im implantierten Zustand an einem Annulus in einer schematischen Darstellung,
- Fig. 10: eine schematische Darstellung einer mechanischen Stützstruktur für die Klappensegel mit Fixiermöglichkeit auf einer proximalen Ringstruktur,
- Fig. 11: ein weiteres alternatives Implantat im implantierten Zustand an einem Annulus in einer schematischen Darstellung,
- Fig. 12A: eine Implantationsvorrichtung bei einer Implantation eines vierten alternativen Implantats in einer schematischen Darstellung und
- Fig. 12B: die Implantationsvorrichtung der Fig. 12A bei einer Aufweitung eines Hohlraums des Implantats der Fig. 12A in einer schematischen Darstellung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verwiesen.

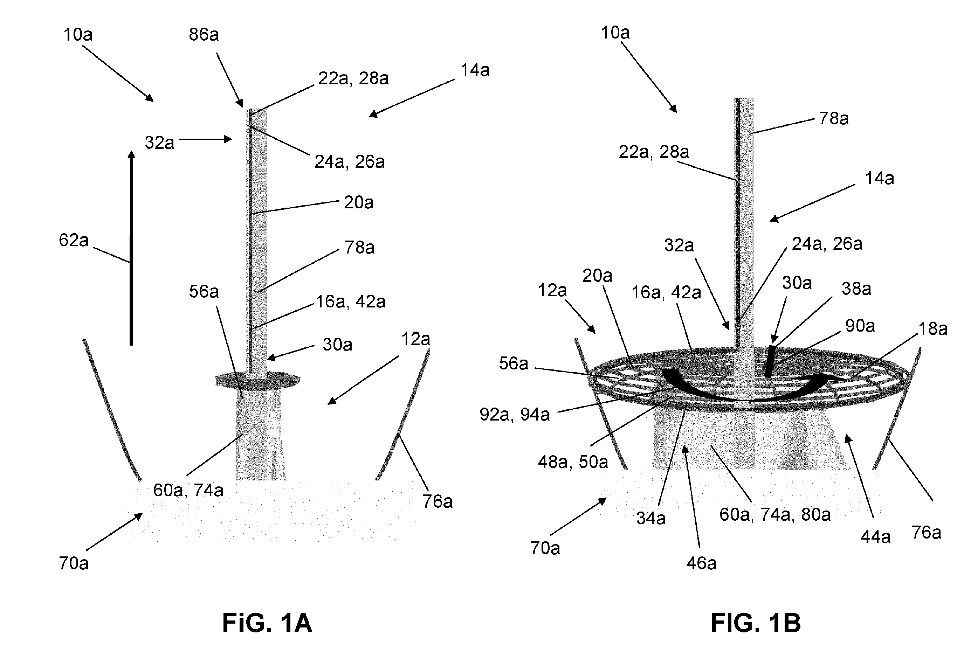

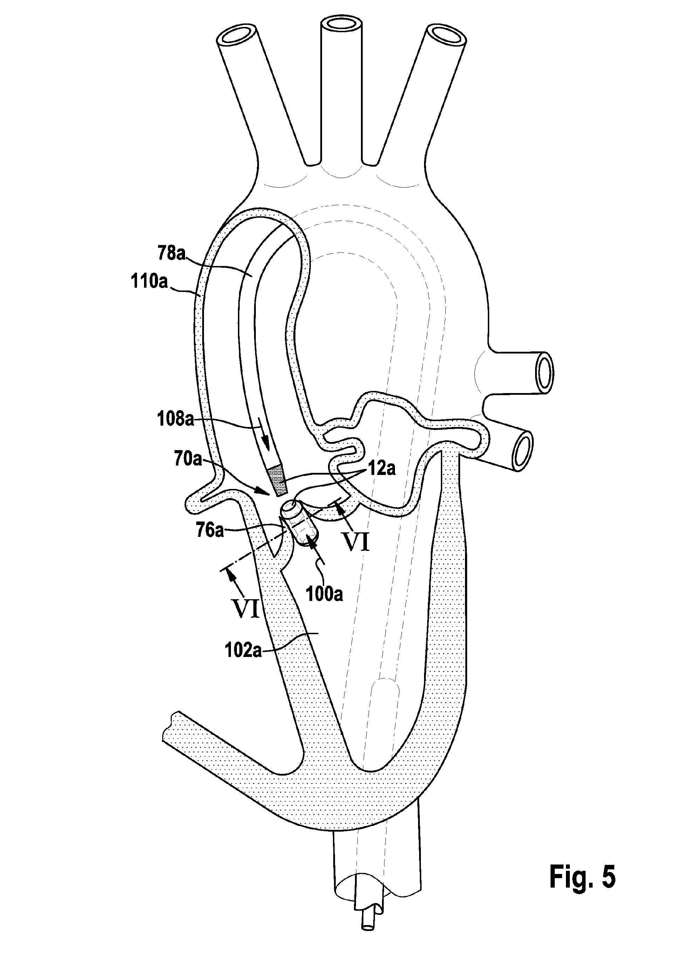

Fig. 1A zeigt eine Implantationsvorrichtung 10a mit einem medizinischen Implantat 12a zur Implantation im tierischen und/oder menschlichen Körper und einer Implantationshilfe 14a. Bei einer Implantation des Implantats 12a an einer Implantationsstelle 70a, wie beispielsweise einem Annulus 76a bzw. einem Aortenannulus, wird die Implantationsvorrichtung 10a mittels eines Kathetersystems 78a in einer dem Fachmann bekannten Weise der Implantationsstelle 70a zugeführt (siehe Fig. 5). Hierbei befindet sich das Implantat 12a, das von einem Klappenimplantat 80a gebildet ist und im implantierten Zustand als künstliche Klappe 60a bzw. Aortenklappe 74a fungiert, in einem kollabierten Zustand. Eine Expansion des Implantats 12a erfolgt mit Hilfe der Implantationshilfe 14a, die sich im Kathetersystem 78a befindet. Hierzu weist die Implantationshilfe 14a ein Zuführelement 22a und ein Aufweitelement 16a auf. Das Aufweitelement 16a weist einen Körper 34a auf, der sich zwischen einem distalen Ende 30a und einem proximalen Ende 32a des Aufweitelements 16a erstreckt. Das Zuführelement 22a und das Aufweitelement 16a bzw. der Körper 34a sind beide als Festkörper 20a ausgebildet bzw. von einem elastischen Draht 28a, 42a bzw. einem elastischen Metalldraht aus Nitinol gebildet.

Wie in der Fig. 1B zu sehen ist, wird das Aufweitelement 16a während der Implantation mittels des Zuführelements 22a in einen Hohlraum 18a des Implantats 12a eingeführt, wobei der Hohlraum 18a dazu ausgebildet ist, im bestimmungsgemäßen Endzustand, welcher den implantierten Zustand darstellt, das Aufweitelement 16a aufzunehmen. Der Hohlraum 18a ist in einem Element 48a eines Grundkörper 46a des Implantats 12a angeordnet. Das Element 48a des Grundkörpers 46a ist von einer Ringstruktur 56a in der Form eines hohlen Rings, gebildet. Ein Bereich 50a bzw. eine Wand oder Umhüllung 82a des Elements 48a/der Ringstruktur 56a ist aus einem Polymer, wie PET, gefertigt und führt bei der Expansion des Aufweitelements 16a dieses. Durch Vorschieben des Aufweitelements 16a in den Hohlraum 18a nimmt der Draht 42a durch die Führung in der Ringstruktur 56a eine elliptische Form an. Der Draht 42a bzw. der Körper 34a wird dadurch reversibel zu einer Feder 44a bzw. Ringfeder umgeformt, welche ausgeprägte Radialkräfte ausübt und die Ringstruktur 56a bzw. den Bereich 50a bzw. die Umhüllung 82a ausdehnt. Dadurch wird der Hohlraum 18a aufgeweitet. Durch die Aufweitung wird eine Fixierung der Ringstruktur 56a an einer Herz- oder Gefäßwand erreicht, wobei dies mit einer selbständigen Anpassung an anatomische Gegebenheiten erfolgt. Optional ist auch eine Ausführung des Hohlraums 18a möglich, welche eine teilweise Überlappung der beiden Enden 30a, 32a des Aufweitelements 16a bei geringem Abstand zueinander vorgibt. Dadurch ist es praktisch möglich bei einer Endfixierung eine Ringform vorzugeben. Ferner lässt sich durch Zurückziehen des Drahts 42a die Fixierung reversibel lösen und das Implantat 12a jederzeit repositionieren.

Nachdem das Implantat 12a an der Implantationsstelle 70a in der richtigen Position fixiert ist, wird das Kathetersystem 78a zurückgezogen. Hierbei weist die Implantationshilfe 14a eine mechanische lösbare Verbindung 24a zwischen dem Zuführelement 22a und dem Aufweitelement 16a auf. Hierbei verbleibt das Aufweitelement 16a im Hohlraum 18a, wodurch das Aufweitelement 16a im bestimmungsgemäßen Endzustand im Hohlraum 18a angeordnet ist.

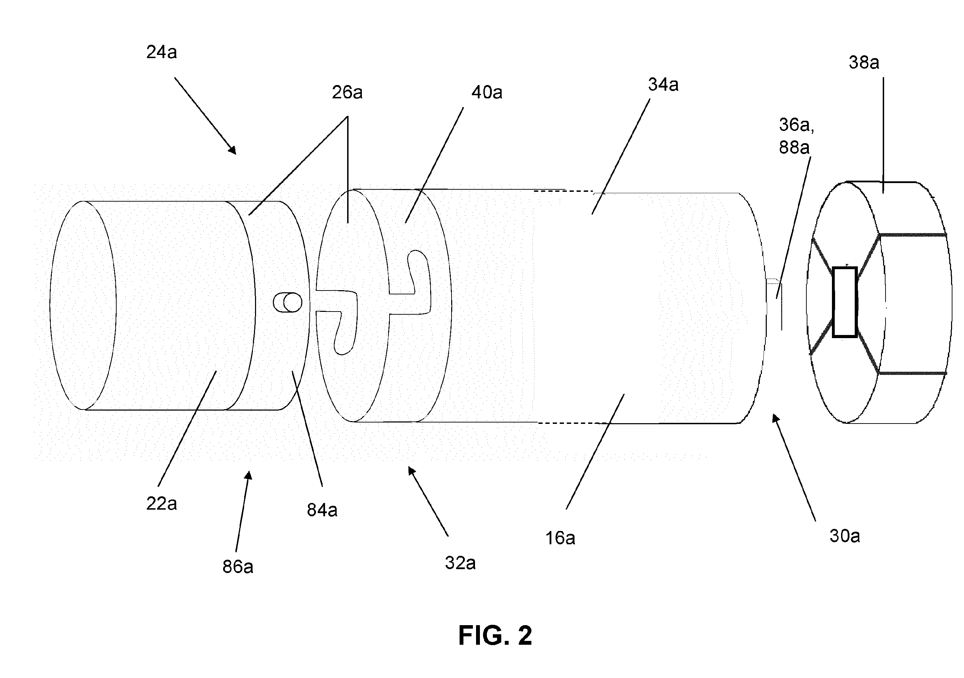

Die lösbare Verbindung 24a ist in Fig. 2 näher dargestellt. Sie weist einen Bajonettverschluss 26a auf, der von einer Verbindungsstruktur 84a des Zuführelements 22a an dessen distalem Ende 86a und einer Verbindungsstruktur 40a des Aufweitelements 16a an dessen proximalem Ende 32a gebildet ist. Wird das Zuführelement 22a gegen den Uhrzeigersinn gedreht, kann die Verbindung 24a geschlossen werden, was eine Manipulation des Aufweitelements 16a erlaubt. Außerdem ist eine Rotation gegen den Uhrzeigersinn um die eigene Achse möglich, was das Vorschieben unterstützen kann. Bei einer Drehung im Uhrzeigersinn kann die Verbindung 24a nach korrekter Platzierung des Implantats 12a geöffnet und das Zuführelement 22a anschließend zurückgezogen werden.

Wie in der Fig. 2 links gezeigt ist, weist das Aufweitelement 16a bzw. der Körper 34a an seinem distalen Ende 30a eine Verbindungsstruktur 36a zur Verbindung mit einem Fixiermittel 38a des Implantats 12a auf. Die Verbindungsstruktur 36a ist als Vierkant 88a ausgebildet und kann nach der erforderlichen Aufweitung und guten Platzierung des Implantats 12a an der Implantationsstelle 70a in dem Fixiermittel 38a, ausgeführt als Gegenstück des Vierkants 88a, verankert werden. Dieses Fixiermittel 38a ist in einer Wand 90a am Ende des Hohlraums 18a angeordnet (siehe Fig. 1B). Eine Ebene dieser Wand 90a erstreckt sich senkrecht zu einer Umfangsrichtung 92a der Ringstruktur 56a. Die Wand 90a ist an einer Seite des Zuführbereichs des Aufweitelements 16a angeordnet, die entgegen der Vorschubrichtung 94a des Aufweitelements 16a orientiert ist. Durch diese Anordnung bildet das Aufweitelement 16a nach der Fixierung im Fixiermittel 38a bzw. im implantierten Zustand einen sich über mindestens 360° erstreckenden, fast geschlossenen Ring.

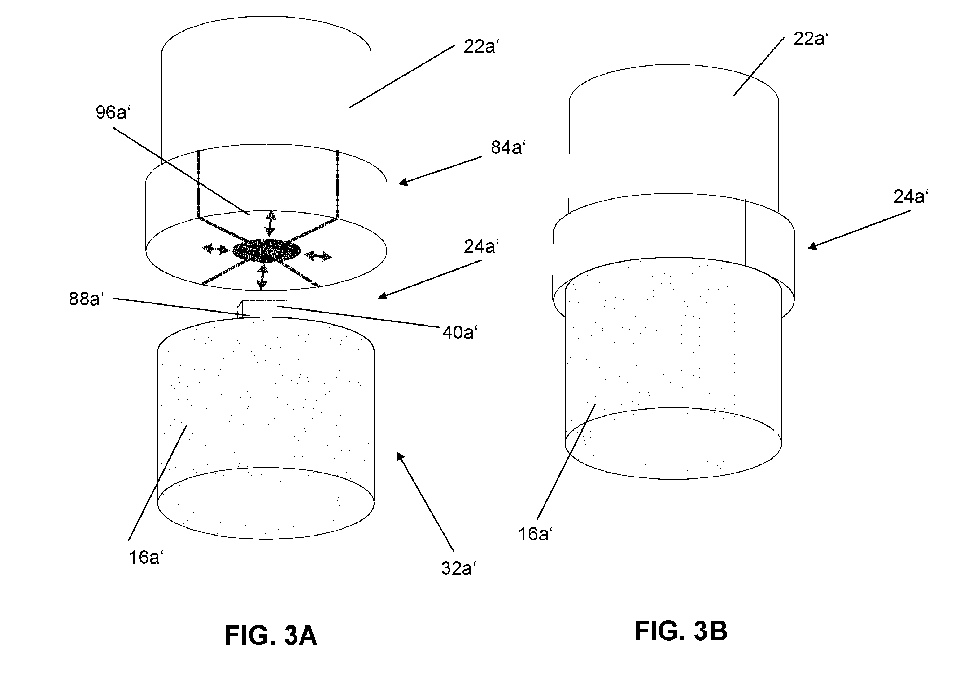

In den Figuren 3A und 3B ist ein alternatives Ausführungsbeispiel der lösbaren Verbindung 24a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele ist jedoch den Bezugszeichen des Ausführungsbeispieles der Figuren 3A, 3B ein Strich hinzugefügt. Die nachfolgende Beschreibung beschränkt sich im Wesentlichen auf die Unterschiede zu dem Ausführungsbeispiel in den Figuren 1 und 2, wobei bezüglich gleich bleibender Bauteile, Merkmale und Funktionen auf die Beschreibung des Ausführungsbeispiels in den Figuren 1 und 2 verwiesen werden kann.

Fig. 3A zeigt eine alternative Ausführung einer reversiblen bzw. lösbaren mechanischen Verbindung 24a' zwischen einem Zuführelement 22a' und einem Aufweitelement 16a'. Hierbei weist ein proximales Ende 32a' des Aufweitelements 16a' als Verbindungsstruktur 40a' einen Vierkant 88a' auf. Passend hierzu sind an einem distalen Ende 86a' des Zuführelements 22a' als Verbindungsstruktur 84a' Spannbacken 96a' angeordnet. Diese lassen sich beispielsweise durch Drehbewegung des Zuführelements 22a' öffnen bzw. schließen (siehe Fig. 3B).

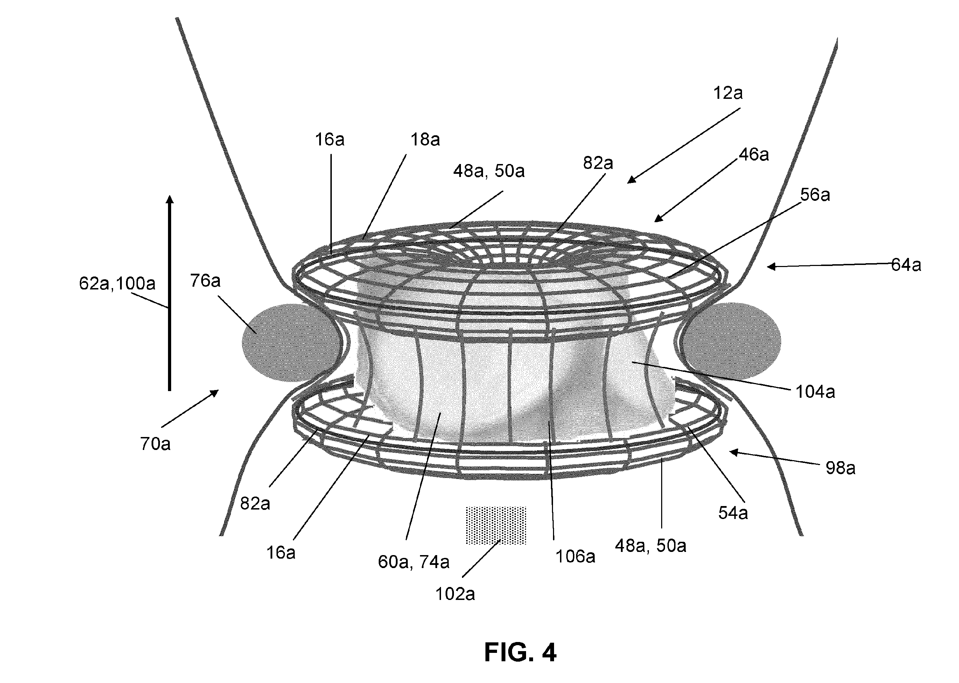

In der Fig. 4 ist das vollständige Implantat 12a nach seiner Implantation an der Implantationsstelle 70a bzw. am Annulus 76a gezeigt. Das Implantat 12a weist zwei Ringstrukturen 54a, 56a auf, die an einem distalen Ende 98a und an einem proximalen Ende 64a des Grundkörpers 46a angeordnet sind. Beide Ringstrukturen 54a, 56a sind unabhängig voneinander mittels eines Aufweitelements 16a aufweitbar. Es wird zuerst die distale Ringstruktur 54a über den entsprechenden Mechanismus im Kathetersystem 78a ausgedehnt und optimal positioniert. Erst wenn die distale Ringstruktur 54a korrekt sitzt, wird die proximale Ringstruktur 56a analog ausgedehnt und positioniert, so dass eine reversible Fixierung/Repositionierung der perkutanen Aortenklappe 74a erfolgt. Somit dienen die Ringstrukturen 54a, 56a als Positioniermittel. Zu einer dauerhaften Fixierung können die Ringstrukturen 54a, 56a mit einem aushärtbaren Material, wie einer Polymerlösung, befüllt werden.

Im bestimmungsgemäßen Endzustand, d.h. im implantierten Zustand, sind die beiden Ringstrukturen 54a, 56a in Fließrichtung 100a eines Fließmediums 102a, wie Blut, in einer axialen Richtung 62a vor und hinter dem Annulus 76a angeordnet. Die Klappe 60a, die Segel 104a aus natürlichem, tierischem Material aufweist, ist in axialer Richtung 62a zwischen den Ringstrukturen 54a, 56a platziert. Durch diese Ausgestaltung lässt sich eine kurze Bauhöhe des Grundkörpers 46a realisieren, wodurch Ausgänge der Koronararterien nicht abgedeckt werden (nicht gezeigt).

Der Grundkörper 46a weist zudem eine Verbindungsstruktur 106a auf, die als ein Hohlzylinder bzw. durchgängige Polymerfolie ausgeführt ist und die beiden Ringstrukturen 54a, 56a miteinander verbindet. Ferner liegt die Verbindungsstruktur 106a in Umfangsrichtung 92a an dem Annulus 76a bzw. den defekten physiologischen Klappen an und verhindert so einen Kontakt der kranken Klappe und der implantierten Klappe 60a. Die Polymerfolie der Verbindungsstruktur 106a kann aus einem aushärtbaren Material gefertigt sein, so dass im ausgehärteten und implantierten Zustand die Verbindungsstruktur 106a einen Stent bilden kann.

In der Fig. 5 ist das Einführen des Implantats 12a schematisch in einer Teilschnittdarstellung illustriert. Die Implantationsvorrichtung 10a mit dem Implantat 12a werden in an sich bekannter Weise an die Implantationsstelle 70a, z.B. den Annulus 76a der natürlichen Aortenklappe mit Segeln, zugeführt. Hierbei ist eine Implantationsrichtung 108a entgegengesetzt zu der Fließrichtung 100a des Fließmediums 102a. Das implantierte Implantat 12a ist gestrichelt angedeutet.

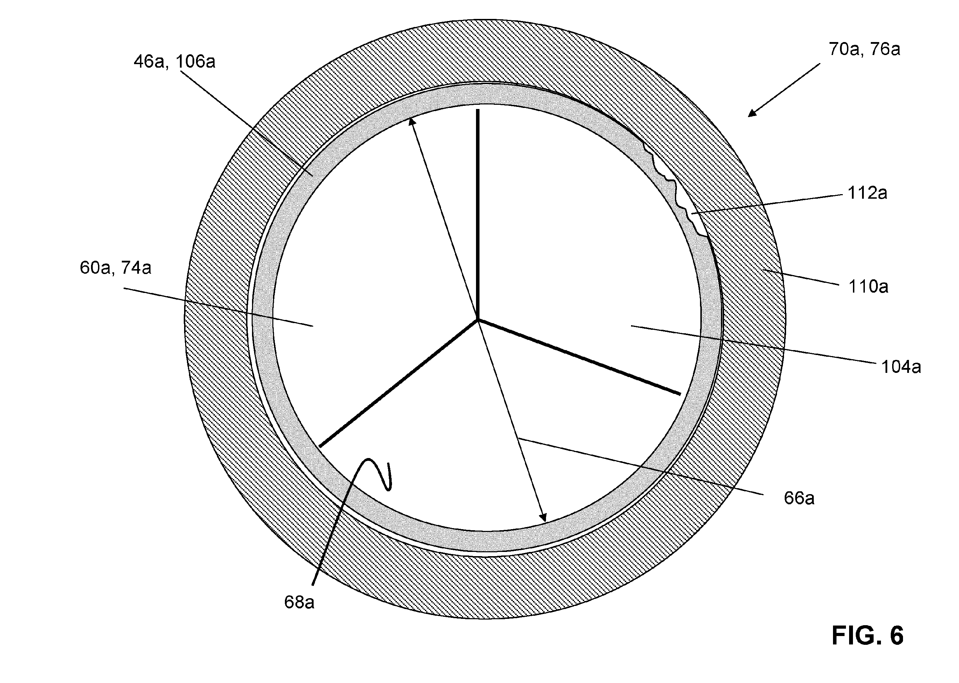

Wie aus der Fig. 6, die einen Schnitt entlang der Linie VI-VI der Fig. 5 durch eine Aortenwand 110a mit Aufsicht auf die Segel 104a der Klappe 60a zeigt, hervorgeht, ist der Grundkörper 46a dazu vorgesehen, einen Unterschied in einer Form, insbesondere einer runden Form eines Innenquerschnitts 66a des Grundkörpers 46a und einer beispielsweise unregelmäßigen Querschnittsfläche 68a der Implantationsstelle 70a auszugleichen. Im Bereich einer Kalzifizierung 112a an der Aortenwand 110a kann sich der Grundkörper 46a während der Implantation durch seine flexible und verformbare Ausgestaltung an die Kontur der Kalzifizierung 112a anpassen, ohne dass eine runde Geometrie der Klappe 60a beeinflusst wird. Dadurch können sich die Segel 104a unbehindert öffnen und schließen. Diese Anpassung kann im Bereich der Ringstrukturen 54a, 56a analog erfolgen.

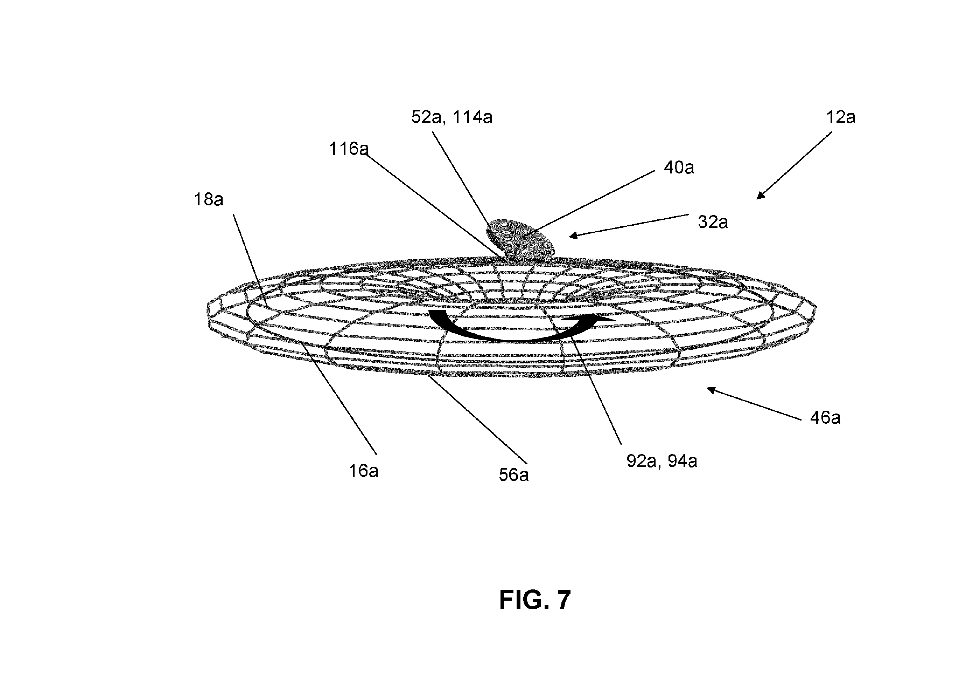

Fig. 7 zeigt ein Führungsmittel 52a, das dazu vorgesehen ist, das Aufweitelement 16a beim Einführen und/oder Aufweiten des Bereichs 50a tangential zu führen. Hierbei bestimmt eine Orientierung bzw. Form des Führungsmittels 52a, in welche Richtung, nämlich die Vorschubrichtung 94a, das Aufweitelement 16a bewegt bzw. gelenkt wird. Das Führungsmittel 52a ist von einem Trichter 114a gebildet und an einem proximalen Ende der jeweiligen Ringstruktur 54a, 56a (es ist hier nur Ringstruktur 56a gezeigt) angeordnet. Zum Einführen in den Hohlraum 18a weist die Ringstruktur 56a eine Aussparung 116a auf. Das proximale Ende 32a des Aufweitelements 16a ragt hierbei im implantierten Zustand mit seinem Verbindungsstruktur 40a aus der Führungsstruktur 52a heraus und verschließt so die Aussparung 116a (hier nicht maßstabsgetreu gezeigt).

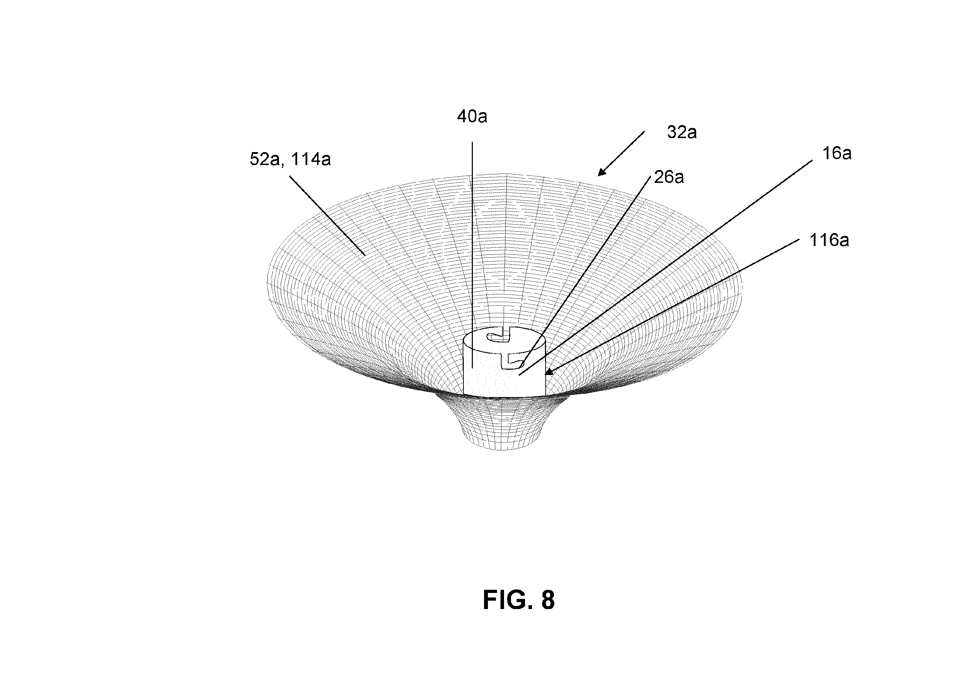

In der Fig. 8 ist eine vergrößerte Darstellung der Anordnung von Führungsmittel 52a und Aufweitelement 16a im eingeschobenen Zustand des Aufweitelements 16a gezeigt. Nach erfolgter Implantation schließt der Bajonettverschluss 26a des Aufweitelements 16a die Aussparung 116a ab. (Der Trichter 114a ist hier nicht maßstabsgetreu gezeigt.)

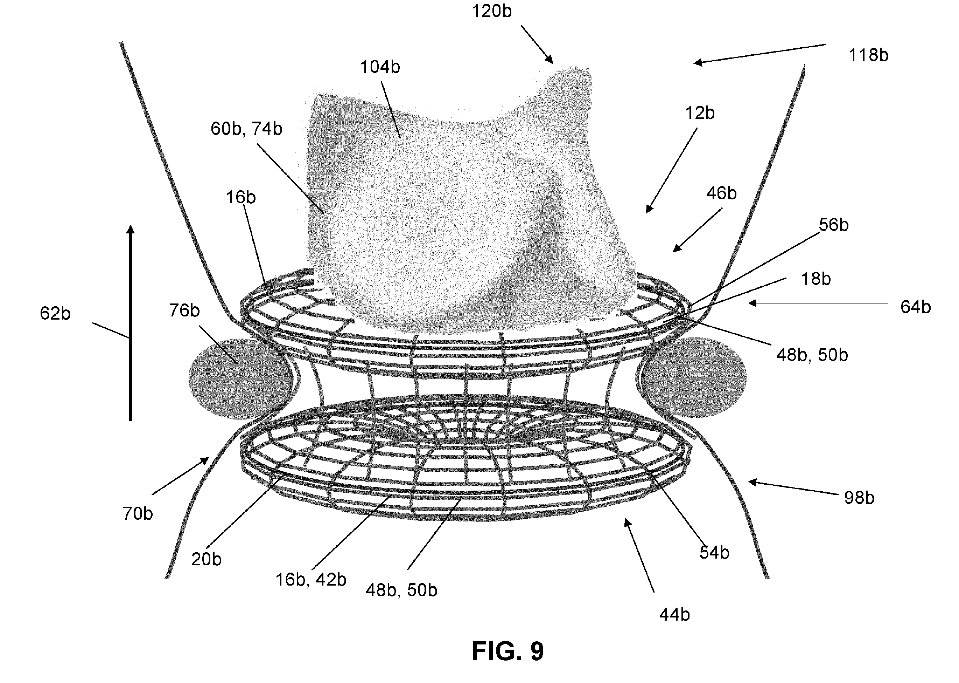

In den Figuren 9 bis 12 sind alternative Ausführungsbeispiele des Implantats 12a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele sind jedoch den Bezugszeichen der Ausführungsbeispiele die Buchstaben a bis d hinzugefügt. Die nachfolgende Beschreibung beschränkt sich im Wesentlichen auf die Unterschiede zu dem Ausführungsbeispiel in den Figuren 1 bis 8, wobei bezüglich gleich bleibender Bauteile, Merkmale und Funktionen auf die Beschreibung des Ausführungsbeispiels in den Figuren 1 bis 8 verwiesen werden kann.

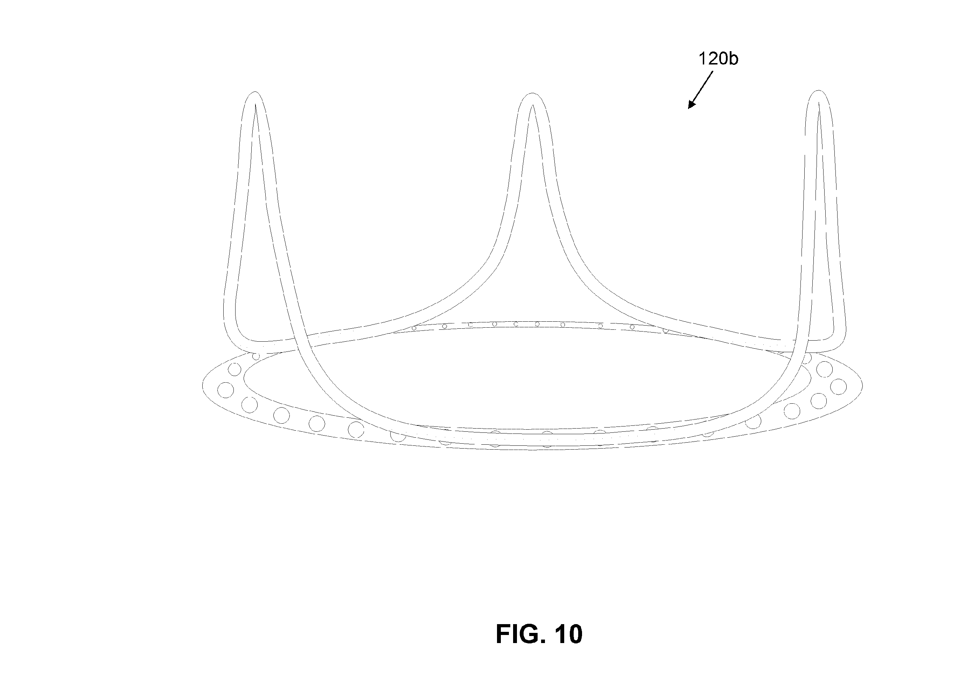

Fig. 9 zeigt ein mittels einer Implantationsvorrichtung (nicht gezeigt) implantierbares alternatives Implantat 12b zur Implantation im tierischen und/oder menschlichen Körper mit einem Grundkörper 46b, der zwei Ringstrukturen 54b, 56b aufweist, die an einem distalen Ende 98b und einem proximalen Ende 64b des Grundkörpers 46b und in axialer Richtung 62b über und unter einer Implantationsstelle 70b bzw. einem Annulus 76b angeordnet sind. Auch hier sind beide Ringstrukturen 54b, 56b aus Elementen 48b aufgebaut, die einen ausdehnbaren Bereich 50b und einen durch ein als eine Feder 44b wirkendes Aufweitelement 16b, bestehend aus einem Festkörper 20b bzw. elastischen Draht 42b, aufweitbaren Hohlraum 18b aufweisen. Eine Klappe 60b, ausgeführt as Aortenklappe 74b, ist in axialer Richtung 62b proximal über der Ringstruktur 56b an dem proximalen Ende 64b des Grundkörpers 46b in der Aorta über dem Annulus 76b angeordnet und an der Ringstruktur 56b fixiert. Diese Anordnung der Elemente 54b, 56b und 60b hintereinander verringert einen Durchmesser des Implantats 12b bei der Implantation. Zu einer oberen Fixierung von Segeln 104b der Klappe 60b ist an der proximalen Ringstruktur 56b bzw. an einem proximalen Ende 118b der Klappe 60b eine mechanische Stützstruktur 120b in der Form eines Metallgerüsts aus z.B. Nitinol befestigt (siehe Fig. 10). Diese stellt die relative Positionierung der Ringstruktur 56b und der Segel 104b sicher und gewährleistet das sichere Öffnungs- und Schließverhalten der Segel 104b.

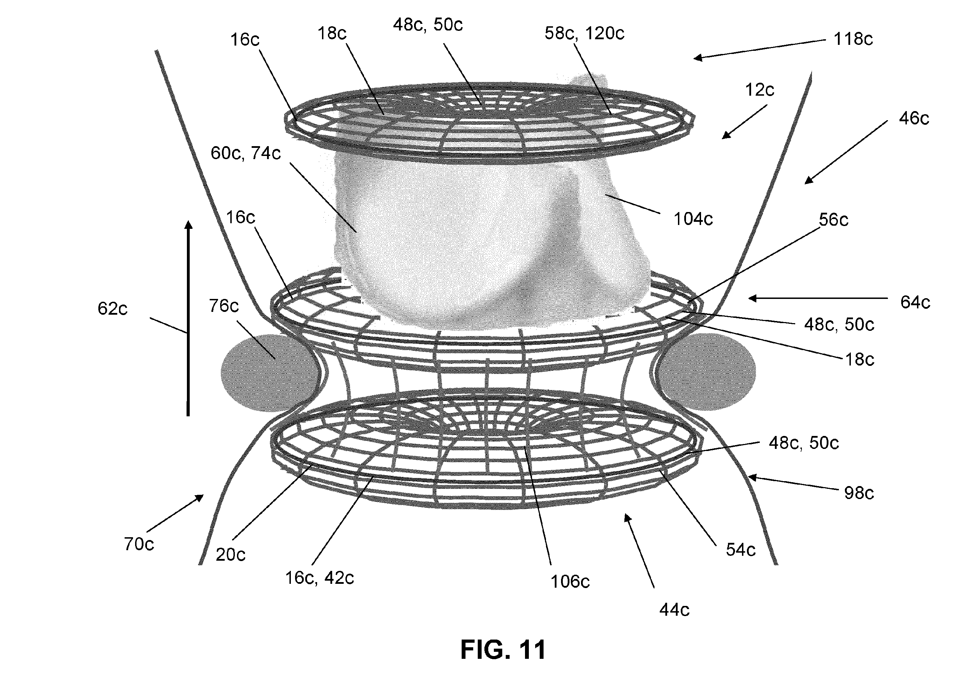

In der Fig. 11 ist ein drittes alternatives Implantat 12c zur Implantation im tierischen und/oder menschlichen Körper mit einer künstlichen Klappe 60c bzw. Aortenklappe 74c und mit einem Grundkörper 46c gezeigt. Die Klappe 60c ist in einer axialen Richtung 62c über einer Ringstruktur 56c an einem proximalen Ende 64c des Grundkörpers 46c in der Aorta über einer Implantationsstelle 70c bzw. einem Annulus 76c angeordnet. Zu einer oberen Fixierung von Segeln 104c der Klappe 60c ist an einem proximalen Ende 118c der Klappe 60c eine mechanische Stützstruktur 120c in der Form einer dritten Ringstruktur 58c angeordnet. Diese Ringstruktur 58c bzw. eine Element 48c ist baugleich zu Ringstrukturen 54c, 56c an dem proximalen Ende 64c und einem distalen Ende 98c des Grundkörpers 46c ausgeführt. Ferner kann sie bzw. ein Bereich 50c und ihr Hohlraum 18c mittels desselben Federringmechanismus wie die Ringstrukturen 54c, 56c durch ein als eine Feder 44c wirkendes Aufweitelement 16c, das aus einem Festkörper 20c bzw. elastischen Draht 42c besteht, ausgedehnt werden. Ferner kann zwischen der Ringstruktur 58c und einer oder beiden Ringstrukturen 54c, 56c eine Verbindungsstruktur analog einer Verbindungsstruktur 106c zwischen den Ringstrukturen 54c, 56cvorgesehen sein (nicht gezeigt).

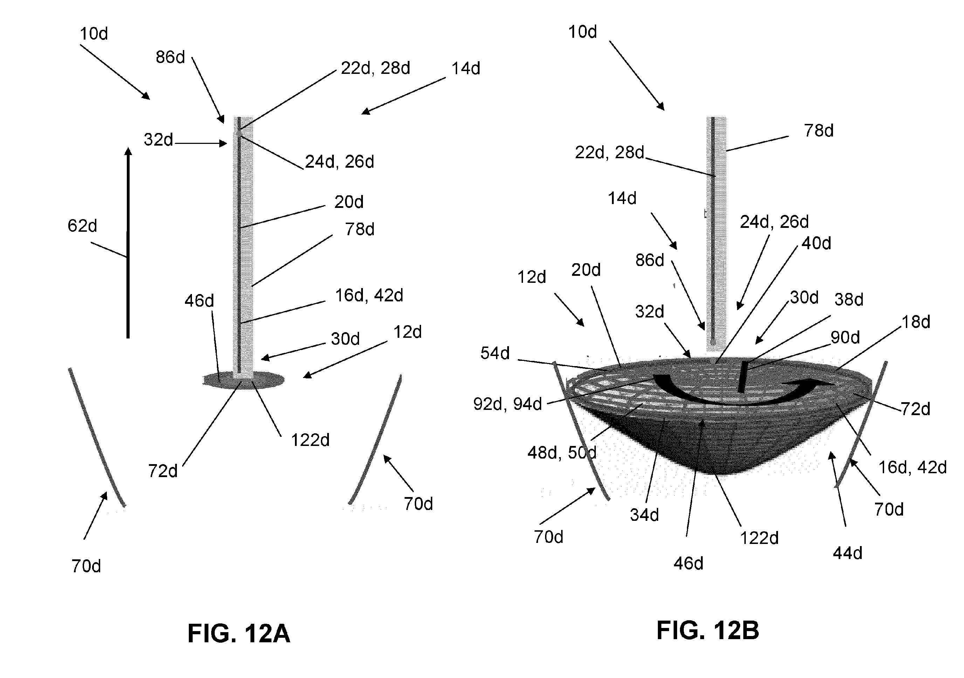

Die Fig. 12A zeigt eine analog zur Implantationsvorrichtung 10a ausgeführte Implantationsvorrichtung 10d mit einem Implantat 12d und einer in einem Kathetersystem 78d angeordneten Implantationshilfe 14d, bestehend aus einem Zuführelement 22d und einem Aufweitelement 16d, die mittels einer lösbaren Verbindung 24d, ausgeführt als Bajonettverschluss 26d, verbunden sind. Das Implantat 12d, das vollständig aus einem als Ringstruktur 54d ausgeführten Grundkörper 46d besteht, ist mittels des in einen Hohlraum 18d des Grundkörpers 46d des Implantats 12d einführbaren Aufweitelements 16d, das von einem Festkörper 20d bzw. einem elastischen Draht 42d gebildet ist, aufweitbar und an einer Implantationsstelle 70d implantierbar (siehe Fig. 12B).

Ferner ist das Implantat 12d als eine Schutzstruktur 72d zu einer temporären Implantation als Schutzeinrichtung (ein sogenanntes "protection device") ausgestaltet. Hierfür weist es an seinem distalen Ende 98d ein Rückhaltemittel 122d auf, das von einem Schirm aus einem Polymernetz gebildet wird. Das protection device lässt sich mit dem Kathetersystem 78d in gefaltetem Zustand in das Zielgefäß führen. Durch Vorschieben des Zuführelements 22d wird die Ringstruktur 54d aufgeweitet und das als protection device fungierende Polymernetz entfaltet. Durch Lösen des Bajonettverschlusses 26d verbleibt das device im Gefäß und der Katheter kann zurückgezogen werden. Das Entfernen des protection device ist mit Durchführung der Schritte in umgekehrter Reihenfolge möglich.

### Bezugszeichenliste

- 10: Implantationsvorrichtung
- 12: Implantat
- 14: Implantationshilfe
- 16: Aufweitelement
- 18: Hohlraum
- 20: Festkörper
- 22: Zuführelement
- 24: Verbindung
- 26: Bajonettverschluss
- 28: Draht
- 30: Ende
- 32: Ende
- 34: Körper
- 36: Verbindungsstruktur
- 38: Fixiermittel
- 40: Verbindungsstruktur
- 42: Draht
- 44: Feder
- 46: Grundkörper
- 48: Element
- 50: Bereich
- 52: Führungsmittel
- 54: Ringstruktur
- 56: Ringstruktur
- 58: Ringstruktur
- 60: Klappe
- 62: Richtung
- 64: Ende
- 66: Innenquerschnitt
- 68: Querschnittsfläche
- 70: Implantationsstelle
- 72: Schutzstruktur
- 74: Aortenklappe
- 76: Annulus
- 78: Kathetersystem
- 80: Klappenimplantat
- 82: Umhüllung
- 84: Verbindungsstruktur
- 86: Ende
- 88: Vierkant
- 90: Wand
- 92: Umfangsrichtung
- 94: Vorschubrichtung
- 96: Spannbacke
- 98: Ende
- 100: Fließrichtung
- 102: Fließmedium
- 104: Segel
- 106: Verbindungsstruktur
- 108: Implantationsrichtung
- 110: Aortenwand
- 112: Kalzifizierung
- 114: Trichter
- 116: Aussparung
- 118: Ende
- 120: Stützstruktur
- 122: Rückhaltemittel

## Patentansprüche

1. Implantationsvorrichtung (10a-d) mit zumindest einem medizinischen Implantat (12a-d) und zumindest einer Implantationshilfe (14a-d) mit zumindest einem Aufweitelement (16a-d), **dadurch gekennzeichnet, dass** das Aufweitelement (16a-d) in zumindest einen Hohlraum (18a-d) des Implantats (12a-d) einführbar ist und der zumindest eine Hohlraum (18a-d) mittels des Aufweitelements (16a-d) aufweitbar ist und das Aufweitelement (16a-d) im bestimmungsgemäßen Endzustand zumindest teilweise in dem zumindest einen Hohlraum (18a-d) angeordnet ist, wobei das Aufweitelement (16a-d) als ein Festkörper (20a-d) ausgebildet ist.

2. Implantationshilfe (14a-d) mit zumindest einem Zuführelement (22a-d) und zumindest einem Aufweitelement (16a-d) für eine Implantationsvorrichtung (10a-d) nach Anspruch 1, **gekennzeichnet durch** zumindest eine lösbare Verbindung (24a-d) zwischen dem zumindest einen Zuführelement (22a-d) und dem zumindest einen Aufweitelement (16a-d).

3. Implantationshilfe nach Anspruch 2, **dadurch gekennzeichnet, dass** die lösbare Verbindung (24a-d) einen Bajonettverschluss (26a-d) aufweist.

4. Implantationshilfe nach Anspruch 2 oder 3, **dadurch gekennzeichnet, dass** das Zuführelement (22a-d) von einem elastischen Draht (28a-d) gebildet ist.

5. Aufweitelement (16a-d) mit einem sich zwischen einem distalen Ende (30a-d) und einem proximalen Ende (32a-d) erstreckenden Körper (34a-d), für eine Implantationsvorrichtung (10a-d) nach Anspruch 1, **dadurch gekennzeichnet, dass** an dem distalen Ende (30a-d) eine Verbindungsstruktur (36a-d) zur Verbindung mit einem Fixiermittel (38a-d) eines Implantats (12a-d) und/oder an dem proximalen Ende (32a-d) eine Verbindungsstruktur (40a-d) zur Verbindung mit einem Zuführelement (22a-d) einer Implantationshilfe (14a-d) angeordnet ist.

6. Aufweitelement nach Anspruch 5, **dadurch gekennzeichnet, dass** der Körper (34a-d) von einem elastischen Draht (42a-d) gebildet ist.

7. Aufweitelement nach einem der vorhergehenden Ansprüche, **dadurch gekennzeichnet, dass** der Körper (34a-d) dazu vorgesehen ist, als eine Feder (44a-d) mit ausgeprägten Radialkräften zu wirken.

8. Medizinisches Implantat (12a-d) zur Implantation im tierischen und/oder menschlichen Körper mit einem Grundkörper (46a-d), der zumindest ein Element (48a-d) mit zumindest einem Hohlraum (18a-d) aufweist, für eine Implantationsvorrichtung (10a-d) nach Anspruch 1, **dadurch gekennzeichnet, dass** der Hohlraum (18a-d) dazu ausgebildet ist, im bestimmungsgemäßen Endzustand ein Aufweitelement (16a-d) aufzunehmen und zumindest ein Bereich (50a-d) des zumindest einen Elements (48a-d) dazu ausgebildet ist, mit dem Aufweitelement (16a-d) aufweitbar ausgestaltet zu sein, wobei das Aufweitelement (16a-d) im bestimmungsgemäßen Endzustand zumindest teilweise in dem zumindest einen Hohlraum (18a-d) angeordnet ist und als ein Festkörper (20a-d) ausgebildet ist.

9. Medizinisches Implantat nach Anspruch 8, **gekennzeichnet durch** zumindest ein Führungsmittel (52a-d), das dazu vorgesehen ist das Aufweitelement (16a-d) beim Einführen und/oder Aufweiten des Bereichs (50a-d) tangential zu führen.

10. Medizinisches Implantat nach Anspruch 8 oder 9, **gekennzeichnet durch** zumindest ein Fixiermittel (38a-d), das in dem zumindest einen Hohlraum (18a-d) angeordnet ist und dazu vorgesehen ist, das Aufweitelement (16a-d) zumindest im bestimmungsgemäßen Endzustand zu fixieren.

11. Medizinisches Implantat nach einem der Ansprüche 8 bis 10, **dadurch gekennzeichnet, dass** das Element (48a-d) des Grundkörpers (46a-d) von zumindest einer Ringstruktur (54a-d, 56a-c, 58c) gebildet ist.

12. Medizinisches Implantat nach Anspruch 11, **gekennzeichnet durch** eine Klappe (60b, 60c), die in einer axialen Richtung (62b, 62c) proximal über der Ringstruktur (56b, 56c) an einem proximalen Ende (64b, 64c) des Grundkörpers (46b, 46c) angeordnet ist.

13. Medizinisches Implantat nach einem der Ansprüche 8 bis 12, **dadurch gekennzeichnet, dass** der Grundkörper (46a-d) dazu vorgesehen ist, einen Unterschied in einer Form eines Innenquerschnitts (66a-d) des Grundkörpers (46a-d) und einer Querschnittsfläche (68a-d) einer Implantationsstelle (70a-d) auszugleichen.

14. Medizinisches Implantat nach einem der Ansprüche 8 bis 13, **gekennzeichnet durch** eine Ausgestaltung als eine Schutzstruktur (72d).

15. Medizinisches Implantat nach einem der Ansprüche 8 bis 13, **gekennzeichnet durch** eine Ausgestaltung als Aortenklappe (74a-c).
